# Europäisches Patentamt

(19) **European Patent Office**

**Office européen des brevets**

(11) Numéro de publication: **0 186 643**

**B1**

(12) **FASCICULE DE BREVET EUROPÉEN**

(45) Date de publication du fascicule du brevet:
**13.09.89**

(21) Numéro de dépôt: **85870160.0**

(22) Date de dépôt: **19.11.85**

(51) Int. Cl.⁴: **C 12 N 15/00**

(54) Procédé de préparation de plasmides vecteurs capables de transformer un hôte bactérien Escherichia coli et d'y promouvoir et contrôler l'expression d'un ADN hétérologue.

(30) Priorité: **23.11.84 BE 901119**

(43) Date de publication de la demande:
**02.07.86 Bulletin 86/27**

(45) Mention de la délivrance du brevet:
**13.09.89 Bulletin 89/37**

(84) Etats contractants désignés:
**AT BE CH DE FR GB IT LI LU NL SE**

(56) Documents cité:
**EP-A-0 040 922**
**EP-A-0 099 084**
**EP-A-0 138 437**
**WO-A-85/01515**

**PROCEEDINGS OF THE NATIONAL ACADEMY OF SCIENCES OF THE USA, vol. 81, no. 3, février 1984, pages 669-673, Washington, US; M. COURTNEY et al.: "High-level production of biologically active human alpha1-antitrypsin in Escherichia coli"**
**NATURE, vol. 263, 28 octobre 1976, pages 744-748; K.J. MARIANS et al.: "Cloned synthetic lac operator DNA is biologically active"**
**GENE, vol. 3, 1978, pages 123-134, Elsevier/North-Holland Biomedical Press, Amsterdam, NL; C.P. BAHL et al.: "Cloned seventeen-nucleotide-long synthetic lactose operator is biologically active"**
**JOURNAL OF THE MOLECULAR AND APPLIED**

(73) Titulaire: **Région Wallonne représentée par l'Exécutif Régional Wallon, 11, Boulevard de l'Empereur, B-1000 Bruxelles (BE)**

(72) Inventeur: **Jacobs, Paul, Chemin des Crolithes, 119, B-7806 Lanquesaint (BE)**
Inventeur: **Morais Gravador, Alfredo Jaime, Avenue des Cygnes, 21, B-1640 Rhode- st- Genese (BE)**
Inventeur: **Bollen, Alex, Gaasbeekstraat 65, B-1711 Itterbek (BE)**

(74) Mandataire: **Blanchart, André, MINISTERE DE LA REGION WALLONE Direction d'Administration de l'Energie et des Technologies nouvelles Service des Technologies nouvelles Avenue Prince de Liège, 7, B-5100 Namur (BE)**

(56) Documents cité: (suite)
**GENETICS, vol. 2, no. 1, janvier 1983, pages 1-10, Raven Press, New York, US; C. QUEEN: "A vector that uses phage signals for efficient synthesis of proteins in Escherichia coli"**
**NUCLEIC ACIDS RESEARCH, vol. 11, no. 3, 1983, pages 773-787, IRL Press Ltd., Oxford, GB; P.L. deHASETH et al.: "Chemical synthesis and biochemical reactivity of bacteriophage lambda PR promoter"**

## EP 0 186 643 B1

**Description**

La présente invention se rapporte à la préparation de plasmides portant une séquence d'ADN choisie en vue de pouvoir y insérer et en extraire aisément une séquence d'ADN hétérologue de façon à ce que cette dernière soit exprimée dans la bactérie hôte <u>Escherichia coli</u> du plasmide. Elle concerne également les plasmides vecteurs obtenus selon les procédés revendiqués. Elle comprend plus particulièrement la conception d'une séquence nucléotidique représentant une partie du promoteur $P_L$ du bactériophage lambda, une séquence représentant le leader de l'ARN messager transcrit à partir de ce promoteur, séquence choisie pour être optimale pour l'initiation de la traduction dudit ARN messager, enfin, une séquence de reconnaissance de différentes enzymes de restriction de façon à permettre l'insertion et l'excision d'ADN hétérologues en phase derrière le site d'initiation de la traduction.

L'expression d'ADN hétérologues en vue d'y diriger la synthèse de polypeptides dans la bactérie hôte <u>Escherichia coli</u> nécessite l'utilisation de plasmides appelés "vecteurs d'expression". De nombreux vecteurs d'expression ont été décrits dans la littérature. Ils ont tous en commun les caractéristiques de porter un promoteur, soit d'origine bactérienne, soit d'origine phagique, suivi d'un site de fixation des ribosomes et d'un site d'insertion d'un ADN hétérologue. L'ADN hétérologue à exprimer est introduit, soit avec son site propre d'initiation de la traduction, soit en phase derrière un site d'initiation de la traduction porté par le vecteur.

La présente invention a pour objet la préparation de plasmides capables de transformer des bactéries <u>E.coli</u>, et portant un promoteur dont la séquence partiellement synthétique est celle d'un promoteur $P_L$ du bactériophage lambda, transcrivant un messager dont le leader a une séquence choisie pour être optimale pour l'initiation de la traduction et possédant une séquence, chevauchant le site d'initiation de la traduction, telle que l'on puisse la cliver par différentes enzymes de restriction.

Ce procédé de préparation de plasmides conçus pour permettre l'expression d'ADN hétérologues dans la bactérie <u>E.coli</u> selon la présente invention, est caractérisé en ce que le plasmide comprend:

a) un promoteur partiellement synthétique de séquence identique à celle du promoteur $P_L$ du bactériophage lambda.

b) une séquence transcrite a partir du promoteur en question qui soit un leader considéré comme optimal pour l'expression d'un ADN hétérologue que l'on peut insérer en aval de ladite séquence leader.

c) une séquence de reconnaissance des enzymes de restriction <u>Kpnl</u>, <u>Ncol</u> et <u>BamHI</u> à cheval sur le site d'initiation de la traduction.

De plus:

a) le promoteur de séquence identique à celle du promoteur $P_L$ du bactériophage lambda est construit à partir d'un promoteur $P_R$ dont la partie comprise entre le site <u>HindII</u> situé dans la région -35 et le site d'initiation de la transcription est remplacée par un fragment synthétique de séquence identique à la séquence naturelle du $P_L$.

b) la séquence leader transcrite à partir du promoteur en question est longue de 23 bases suivies d'un codon ATG d'initiation de la traduction, elle comprend un site de liaison des ribosomes homologue à l'ARN 16S du ribosome sur 9 bases et situé 12 bases en amont de l'ATG.

c) les séquences reconnues par les enzymes <u>Kpnl</u>, <u>Ncol</u> et <u>BamHI</u> sont combinées en une séquence de longueur minimum qui est:

```
          KpnI                    BamHI
      ┌─────────────┐         ┌──────────── ──┐
      G G T A C C A T G G A T C C
              └──────── ───────┘
                   NcoI
```

De plus:

a) le promoteur $P_L$ et le leader transcrit à partir de ce promoteur sont construits à partir du promoteur $P_R$ du plasmide vecteur pCQV2 par remplacement d'un fragment <u>HindII</u>-<u>BamHI</u> de 56 paires de bases par un fragment de 60 paires de bases synthétisé sous forme de 4 oligodésoxyribonucléotides.

b) des bactéries hôtes <u>E.coli</u> sont transformées par le plasmide obtenu sous a), sélectionnées, et isolées par crible caractéristique, et les vecteurs portés sont caractérisés par séquençage des régions relevantes.

Enfin, la séquence leader inventée est la suivante:

```
A T C A G C T A A G G A G G T T T T G G T A C C A T G
↑                                          ↑
initiation de la                           initiation de
transcription                              la traduction
```

Le procédé faisant l'objet de la présente invention est plus particulièrement caractérisé par le choix des moyens suivants:

a) on conçoit de modifier le vecteur pCQV2 d'usage libre de façon à ce que le promoteur $P_R$ qu'il porte devienne un promoteur $P_L$ en utilisant le fait qu'il existe au sein de la région d'homologie -35 de ces deux promoteurs, un site de restriction <u>HindII</u> et que la région la plus éloignée du site d'initiation de la transcription par rapport au clivage <u>HindII</u> est identique pour les deux promoteurs.

2

b) on conçoit une séquence optimale pour l'initiation de la traduction en prenant celle dont la complémentarité avec l'ARN 165 du ribosome est la plus grande dans les limites de la dimension permise de cette séquence et en faisant suivre cette séquence de 12 bases puis d'un ATG, codon d'initiation de la traduction, la distance de 12 bases entre le site de fixation du ribosome et le site d'initiation de la traduction étant considérée comme optimale.

c) on découpe la séquence ainsi conçue en 4 fragments monobrins convenables pour être synthétisés chimiquement et être clonés en deux étapes distinctes permettant un contrôle intermédiaire du bon fonctionnement du vecteur.

d) on synthétise chimiquement les 4 fragments dont l'ensemble couvre la partie du promoteur $P_L$ comprise entre le site HindII de la région d'homologie -35 et le site d'initiation de la transcription ainsi que le leader de l'ARN messager et la région portant les sites d'insertion choisis.

e) on insère les 4 fragments synthétiques dans le plasmide pCQV2 clivé par les enzymes HindII et BamHI et débarrassé du fragment HindII et BamHI de 56 paires de bases à remplacer.

f) on transforme des bactéries de la souche MM294 par l'ADN hybride obtenu dans la phase précédente.

g) on sélectionne, par leur résistance à un antibiotique, les bactéries qui portent un plasmide.

h) on isole de cette banque les clones porteurs de l'ADN inséré par hybridation avec un fragment constitutif de l'ADN inséré marqué au $^{32}$P.

i) on caractérise le vecteur par séquençage des régions relevantes par la méthode de Maxam & Gilbert, à partir d'un site de restriction proche de l'ADN inséré dans le plasmide. On obtient alors un premier vecteur.

j) on mesure le caractère fonctionnel du plasmide en insérant en phase derrière l'ATG un ADN hétérologue et en mesurant au moyen d'un test ELISA le taux de synthèse de la protéine codée par l'ADN hétérologue.

Suivant une variante du procédé faisant l'objet de la présente invention,

a) on délète le vecteur obtenu d'un fragment HindIII-HindIII de 125 paires de bases situées dans le gène cl codant pour le répresseur des promoteurs $P_R$ et $P_L$.

b) on transforme, on sélectionne et on caractérise le résultat comme décrit plus haut de f) à j). On obtient alors un second vecteur; ce vecteur ne portant plus de répresseur de son propre promoteur devra être utilisé dans une souche lysogène pour un bactériophage λ défectif porteur du gène cl, soit sous sa forme sauvage, soit sous forme d'un mutant thermosensible $cl_{857}$).

Suivant une autre variante du procédé,

a) on insère deux fragments synthétiques complémentaires de 34 bases dans le vecteur obtenu clivé par les enzymes BamHI et PvuII débarrassé du fragment BamHI-PvuII de 1692 paires de bases. La séquence de 34 paires de bases est choisie de façon à présenter un maximum de sites de restriction qui par ailleurs ne se retrouvent pas en une autre localisation dans le vecteur. Elle porte notamment les sites SacII, BalI, SalI, XhoI, SacI et PvuII, et est la suivante:

```
      BamHI        SacII          BalI
    ┌───────┐   ┌──────────┐   ┌─────────┐
    G A T C C   C C G C G G T   G G C C A
      SalI         XhoI     SacI     PvuII
    ┌───────┐   ┌───────────┬─────────┐
    G T C G A C   C T C G A G C T C A G
```

b) on transforme, on sélectionne et on caractérise le résultat comme décrit plus haut de f) à j). On obtient alors un troisième vecteur.

Le procédé de préparation de plasmides d'expression d'ADN hétérologue dans la bactérie E.coli est caractérisé par la combinaison et le choix d'un ensemble de moyens détaillés ci-après.

La construction d'un vecteur d'expression performant peut être faite par exemple en modifiant un vecteur d'expression banal, par exemple pCQV2 décrit par Queen (Mol. App. Genet., 2 (1983)1-10), en vue d'optimaliser les différents éléments influant sur son efficacité. Ces éléments sont principalement le taux de transcription dépendant de la séquence du promoteur et plus particulièrement des régions -35 et -10; ainsi que l'efficacité de l'initiation de la traduction déterminée par la séquence du site de liaison du ribosome et la séquence et la distance entre celui-ci et l'ATG d'initiation. On trouvera dans Rosenberg et Court (Annual Rev. of Genet. 13 1979, pp. 319 - 354) une comparaison de différentes séquences promotrices bactériennes et de bactériophages; dans Kastelein, R.A., Berkhout, B., Overbeek, G.P. et Van Duin, J. (Gene 23 (1983) 245 - 254), Joy, E., Seth, A.K., Romens, J., Sood, A. and Joy, G. (Nucleic Adics Res. 10 (1982) 6319 - 6329), Gheysen, D., Iserentout, D., Derom, C. and Fiers, W. (Gene 17 (1982) 55 - 63), Joy, G., Khoury, G., Seth, A.K. and Joy, E. (Proc. Natl. Acad. Sci. USA 78 (1981) 5543 - 5548), Hui, A., Hoyplick, J., Dinkelspiel, K. and de Boer, H.A. (The EMBO J. 3 (1984) 623 - 629), l'état des connaissances quant aux facteurs influençant l'initiation de la traduction chez E.coli.

On peut concevoir une séquence qui paraisse optimale au vu des différents critères tels qu'ils ressortent de la littérature citée; par exemple:

a) la séquence promotrice sera celle du promoteur $P_L$ du bactériophage lambda;

b) le site de reconnaissance du ribosome sera complémentaire de l'ARN 16S du ribosome sur 9 bases et la distance entre ce site et l'ATG d'initiation sera d'environ 10 paires de bases. En aval de l'ATG d'initiation, on peut concevoir une séquence telle que un maximum de sites de restriction apparaissent sur une distance minimale et que ces sites soient uniques dans le vecteur. Cette séquence peut par exemple être conçue pour commencer par l'extrémité cohésive d'un site BamHI et se terminer par une extrémité franche.

c) on peut créer une telle séquence, par exemple en la découpant en fragments simple brin de façon optimale, compte tenu des possibilités de la synthèse chimique d'ADN que l'on peut trouver décrite dans Narang, S.A. (Tetrahedron 39 (1983) 3 - 22), et de façon à pouvoir réaliser le clonage de ces fragments en deux étapes, un premier groupe de 4 fragments se terminant par exemple par un site BamHI, les deux derniers fragments ayant un site BamHI à leur extrémité 5' sur la fibre codante et une site PvuII à leur extrémité 3' sur la même fibre.

d) la synthèse chimique des fragments d'ADN voulus peut se faire selon différentes méthodes dont on trouvera la description dans Narang, S.A. (Tetrahedron 39 (1983) 3 - 22), Itakura, K. (Trends in Biochem. Sc. (1982) 442 - 445), Ohtruka, E., Ikehara, M. and Soll, D. (Nucleic Acids Res. 10 (1982) 6553 - 6570). A titre d'exemple, on peut utiliser soit la méthode des phosphotriesters décrite par Brown, E.L. Belagage, R., Ryan, M.J. and Khorama, M.G. (Methods in Enzymology 68 (1979) 109), soit celle des phosphites triesters décrite par Letsinger, R.L. and Lusford, W.B. (J. Am. Chem. Soc. 98 (1976) 3655). Ces méthodes diffèrent par le degré d'oxydation de l'atome de phosphore dans les intermédiaires nucléotidiques mis en oeuvre lors de la réaction de couplage.

e) L'hybridation et la ligation des fragments d'ADN synthétique entre eux peut se faire par différentes méthodes dont on trouvera la description dans Molecular Cloning (1982 - Maniatis, T., Fritsch, E.F. and Sambrook, J., CS Publ.). Ces méthodes sont basées sur le principe de l'hybridation des séquences complémentaires d'ADN. Elles consistent à mélanger les fragments d'ADN dans des conditions de température et de force ionique adéquates à la formation des hybrides spécifiques. La ligation de ces fragments hybridés est le résultat de l'action d'une enzyme, par exemple la T4 DNA ligase. La formation in vitro de molécules hybrides entre l'ADN synthétique double brin à cloner et l'ADN de vecteurs plasmides s'effectue selon le procédé décrit dans Molecular Cloning (1982 - Maniatis, T., Fritsch, E.F. and Sambrook, J., CSH Publ.), qui consiste à mélanger les molécules dans des conditions de température et de force ionique appropriées. C'est ce protocole qui a été suivi dans la réalisation de l'invention. L'ADN vecteur employé dans la réaction de recircularisation in vitro peut provenir de différents plasmides. Dans la réalisation de cette invention, on choisit de préférence le plasmide pCQV2 (Queen, C., J. Mol. Applied Genet. 2 (1983) 1 - 10) dans lequel on clone entre les sites HindII et BamHI d'une part, et BamHI et PvuII d'autre part.

f) La plupart des méthodes utilisées pour transformer des bactéries par de l'ADN exogène font appel à un traitement particulier des bactéries par du chlorure de calcium, et visent à optimiser l'efficacité de transformation pour différentes souches de bactéries. Dans le cadre de l'invention, plusieurs types de bactéries peuvent servir d'hôtes aux vecteurs utilisés. On prendra de préférence la souche d'Escherichia coli MM294 pour les vecteurs portant leur propre répresseur, et N99 et N5151 pour les vecteurs dépourvus de leur propre répresseur. Leurs conditions de croissance sont particulièrement commodes. En outre, parmi les différents procédés de transformation, on choisit celui décrit dans Mandel, M. and Higa, A. (J. Mol. Biol. 53 (1970) 154).

g) En raison des marqueurs de résistance aux antibiotiques présents dans l'ADN du vecteur, il est simple d'isoler les bactéries transformées par l'ADN recombiné in vitro. Par exemple, si l'ADN du vecteur porte le gène $Tet^R$, toute bactérie portant un tel vecteur sera résistante à la tétracycline. En outre, si l'insertion de l'ADN dans le vecteur a pour effet d'inactiver un gène de résistance à un antibiotique, on repérera les bactéries qui portent le vecteur recombinant par leur sensibilité à cet antibiotique. De préférence on choisit comme marqueur de sélection le caractère $Amp^R$ des plasmides construits.

h) Le crible de sélection des clones porteurs de l'ADN-synthétique peut se faire selon des méthodes mettant en oeuvre une sonde synthétique. Cette approche est détaillée dans Genetic Engineering, vol. 1, Williamson, R. Ed., (1981) Academic Press, N.Y. Dans le cadre de la présente invention, on utilise de préférence l'un ou plusieurs des fragments constitutifs de l'ADN-synthétique marqué au $^{32}P$ comme sonde oligodésoxyribonucléotidique que l'on hybride avec les clones de la banque.

i) La séquence d'ADN créée dans l'un ou plusieurs des clones obtenus peut être caractérisée par des méthodes de séquençage décrites dans Maxam, A. & Gilbert, W. (Methods in Enzymology 65 (1980) 497 - 559) et dans Sanger, F., Nicklen, S. & Coulson, A.R. (Proc. Natl. Acad. Sci. USA 74 (1977) 5463). Dans le cadre de la présente invention, on choisira de préférence la méthode de Maxam & Gilbert.

j) On peut alors tester la valeur des plasmides créés, par exemple en y insérant un ADN hétérologue dont l'expression peut être mesurée par exemple par un test ELISA comme décrit dans Bollen, A., Herzog, A., Cravador, A., Hérion, P., Chuchana, P., van der Straten, A., Loriau, R., Jacobs, P. & Van Elsen, A. (DNA 2 (1983) 255 - 264).

L'exemple non limitatif suivant qui s'applique à la construction de vecteurs d'expression à partir du plasmide pCQV2 permettra de mieux comprendre l'invention.

**A. Construction du plasmide vecteur désigné ci-après par pULB1219 et obtenu suivant les revendications 1 à 4.**

1. Modification du promoteur $P_R$ du vecteur pCQV2 en promoteur $P_l$ et optimalisation des séquences influençant l'initiation de la traduction.

Dans le cadre de la présente inventions nous avons choisi de modifier le promoteur $P_R$ du vecteur pCQV2 pour en faire un promoteur $P_L$. Pour cela, nous avons inventé la séquence double brin suivante.

```
        1                  10                  20
   5'G A C A T A A A T A C C A C T G G C G G T G A T A C T
   3'C T G T A T T T A T G G T G A C C G C C A C T A T G A
        60                  50                  40


        30                  40                  50
   G A G C A C A T C A G C T A A G G A G G T T T T G G T
   C T C G T G T A G T C G A T T C C T C C A A A A C C A
                    30                  20



                                  c ú
             ú c c ú t g                3'
             ú ú g t a c c t a g        5'
             ú ú                        1
```

Cette séquence est caractérisée en ce que:

a) son extrémité 5' (considérant la fibre identique au mRNA) est franche et correspond à la coupure par l'enzyme HindII dans la région -35 homologue aux promoteurs $P_R$ et $P_L$, en ce qu'elle est identique à la séquence naturelle du $P_L$ jusqu'au site d'initiation de la transcription (base 34 sur la fibre codante),

b) les 26 bases suivantes sont choisies pour constituer un site de reconnaissance par les ribosomes qui soit optimal pour que la séquence et la distance séparant ce site de reconnaissance du site d'initiation de la traduction (ATG, bases 58, 59, 60) soit optimale, et pour présenter devant cet ATG les sites de reconnaissances des enzymes KpnI et NcoI,

c) son extrémité 3' se termine par la partie d'un site de restriction BamHI telle qu'elle apparaît après clivage d'un ADN par cette enzyme.

Dans le cadre de la présente invention, nous avons choisi de synthétiser cette séquence sous forme de 4 fragments simple brin allant des bases 1 à 27 et 28 à 60 sur la fibre codante et 1 à 30 et 31 à 64 sur la fibre complémentaire.

## 2. Préparation et ligation des fragments constitutifs

Dans le cadre de la présente invention, on choisira de préférence de digérer le plasmide pCQV2 par les enzymes BamHI et HindII et d'isoler par électrophorèse sur gel d'acrylamide et électroélution les fragments obtenus de 4302, 459 et 125 paires de bases. On choisira ensuite de digérer le fragment de 459 paires de bases par l'enzyme HindIII ce qui génère 2 fragments de 403 et 56 paires de bases qui sont séparés par électrophorèse; le fragment de 403 paires de bases est électroélué.

Par ailleurs on choisit de ne phosphoryler que les fragments de 33 et 34 bases de façon à éviter la formation de polymères lors de la ligation. On mélange alors 50 pmoles de chacun des 4 fragments synthétiques; le mélange est porté 2' à 90°C et refroidi lentement jusque 15°C, on ajoute 10 % en volume d'acétate de sodium 3M et 2,5 vol. d'éthanol, on refroidit 20' à -80°C et on centrifuge. Le culot est lavé à l'éthanol 75 % dans l'eau, séché et resuspendu en tampon de ligation (50 mM Tris HCl pH 7.4, 10 mM MgCl$_2$, 10 mM DTT, 1 mM spermidine, 1 mM ATP; 0,1 mg/ml BSA). Les trois fragments de pCQV2 de taille 4302, 403 et 125 également en solution en tampon de ligation, sont ajoutés à raison de 40 pmoles de chaque. Le mélange est incubé 16 h à 15°C en présence de 5 unités de l'enzyme T4 DNA ligase.

## 3. Transformation des bactéries hôtes MM294

On procède alors à la transformation de la souche MM294 ("Molecular Cloning", Maniatis, J., Fritsch, E.F., Sambrook, J., CSH Ed. 1982) dont le système de restriction est modifié de façon à tolérer la présence d'un ADN étranger selon la méthode décrite dans "Molecular Cloning" (Maniatis, J., Fritsch, E.F. and Sambrook, J., CSH Ed. 1982).

## 4. Obtention de la banque de clones

En raison du caractère Amp[R] du plasmide, on peut sélectionner les bactéries transformées par simple croissance sur milieu contenant de l'ampicilline. L'ensemble des manipulations décrites ci-dessus a conduit à l'obtention d'une banque de 100 clones dont une grande partie doit contenir un plasmide de séquence voulue.

## 5. Isolement des clones de séquence voulue

On a recherché les clones porteurs d'un plasmide ayant la séquence voulue au moyen du fragment de 34 bases utilisé comme sonde. Après avoir marqué cette sonde au $^{32}$P par une réaction enzymatique (kination en 5' de la sonde), nous l'avons utilisée pour cribler la banque de clones. Pratiquement, on fixe l'ADN de chaque clone sur feuille de nitrocellulose, suivant la technique de Grunstein, M. et Hogness, D. (Proc. Natl. Acad. Sci. USA 78 (1975) 3961) et on l'hybride avec la sonde synthétique marquée au $^{32}$P dans des conditions ioniques et de température appropriées (0,9 NaCl et 55°C), la sonde radioactive va reconnaître spécifiquement l'ADN des clones portant une séquence homologue, c'est-à-dire une séquence nucléotidique typique du promoteur P$_L$. On visualise les clones positifs par autoradiographie. Les clones positifs sont alors analysés par restriction par les enzymes HindII, BamHI, Ncol, HindIII; à cette étape, un clone ayant toutes les propriétés voulues a été retenu, pULB1219.

## 6. Séquençage du clone pULB1219

On caractérise le plasmide du clone pULB1219 par l'établissement de la séquence nucléotidique de la région modifiée et des jonctions avec les régions non modifiées. A cet effet, l'ADN du plasmide recombinant est préparé et clivé au site de restriction BamHI unique dans le plasmide recombinant. Les extrémités du plasmide linéarisé sont alors marquées au $^{32}$P. On coupe ensuite le plasmide par l'enzyme EcoRI générant deux fragments dont l'un porte à son extrémité marquée la séquence synthétique. Le fragment marqué est alors soumis à une série de réactions chimiques conduisant à la production d'oligonucléotides marqués, de tailles différentes, dont l'analyse sur gel de polyacrylamide conduit à l'établissement de la séquence en bases suivant la technique de Maxam, A.M. & Gilbert, N. (Proc. Natl. Acad. Sci. USA 74 (1977) 560). On voit que la séquence obtenue est parfaitement conforme à celle définie en 1., et jointe aux fragments de pCQV2 de la façon choisie.

**B. Construction du plasmide vecteur désigné ci-après par pULB1220 et obtenu suivant la revendication 5.**

1. L'ADN du plasmide pULB1219 est clivé par l'enzyme de restriction HindIII générant deux fragments. Ceux-ci sont séparés sur gel de polyacrylamide et le plus grand est électroélué.
2. Ce fragment est alors religué sur lui-même générant le plasmide vecteur pULB1220.
3. Le plasmide vecteur pULB1220 est utilisé pour transformer la souche de E.coli N99 lysogène pour un λ défectif portant le gène cl sauvage. Ceci conduit à l'obtention d'une banque de clones caractérisés par simple restriction.

**C. Construction du plasmide vecteur désigné ci-après par pULB1221 et obtenu suivant les revendications 6 à 8.**

### 1. Conception d'une séquence d'insertion/excision d'ADN hétérologue

On choisit, pour l'insérer entre les sites BamHI et Pvull du fragment obtenu, la séquence suivante.

```
         1                      10
    5'  G A T C C C C G C G G T G G C C A
         3'  G G G C G C C A C C G G T
            30                      20

         20                      30
    G T C G A C C T C G A G C T C A G  3'
    C A G C T G G A G C T C G A G T C  5'
                    10                      1
```

présentant notamment les sites de restriction Sacll, Ball, Accl, Sall, Aval, Xhol, HgiAl, Sacl. De plus, l'insertion de cette séquence dans pULB1220 régénère les sites BamHI et Pvull.

2. L'ADN du plasmide vecteur pULB1220 est clivé par les enzymes de restriction BamHI et Pvull. Cette digestion génère deux fragments, qui sont séparés sur gel de polyacrylamide. Le plus grand est purifié par électroélution.

3. La séquence choisie est synthétisée sous forme de 2 fragments monobrins complémentaires comme décrit plus haut.

4. Les 2 fragments monobrins sont hybridés et ligués comme décrit plus haut.

5. Le plasmide vecteur obtenu est utilisé pour transformer la souche N99 comme décrit.

6. La banque de clones obtenue est caractérisée par hybridation avec le fragment de 34 bases utilisé comme sonde, comme décrit. Les clones positifs sont alors testés pour la présence des sites de restriction portés par le fragment synthétique. Le clone pULB1221 est retenu.

7. L'ADN du plasmide vecteur pULB1221 est isolé et clivé par l'enzyme Ncol. Le plasmide linéarisé est marqué au $^{32}$P comme décrit, recoupé par l'enzyme EcoRI et séquencé comme décrit. La séquence est exactement celle choisie.

**D. Test de l'efficacité des plasmides vecteurs pULB1219, pULB1220 et pULB1221.**

1. Afin de tester l'efficacité des plasmides vecteurs pULB1219, pULB1220 et pULB1221, un cDNA codant pour l'$\alpha_1$-antitrypsine humaine ($\alpha$-AT) a été inséré en phase au site BamHI de chacun d'eux ainsi qu'au site BamHI du plasmide vecteur pCQV2; pour cela, un fragment de cDNA de l'$\alpha_1$-AT portant un site BamHI à son extrémité NH$_2$ terminale et une extrémité franche après le stop, a été ligué comme décrit, avec les plasmides pCQV2, pULB1219, pULB1220, clivés par BamHI et PvuII, et pULB1221 clivé par BamHI et Ball. Les plasmides recombinants obtenus ont été utilisés pour transformer les souches MM294 dans les cas de pCQV2 et pULB1219, et N99 dans les cas de pULB1220 et pULB1221. Les transformants ont été caractérisés par extraction à petite échelle de leur ADN plasmidique et restrictions appropriées. Dans le cas des plasmides pULB1220 et pULB1221, l'ADN plasmidique de clones jugés corrects est utilisé pour retransformer la souche N5151 lysogène pour un $\lambda$ défectif porteur d'un gène cI muté thermosensible (cI$_{857}$). Les souches ainsi construites, pCQV2 $\alpha_1$-AT dans MM294, pULB1219 $\alpha_1$-AT dans MM294, pULB1220 $\alpha_1$-AT dans N5151 et pULB1221 $\alpha_1$-AT dans N5151, sont alors cultivées à 32°C jusqu'à une densité optique de 0,5 a 660 nm. La culture est alors divisée en deux parts dont l'une reste à 32°C et l'autre est portée rapidement à 42°C. Après 1 h 30, les cellules sont récoltées par centrifugation puis extraites au lysozyme et Triton X100. L'$\alpha_1$-antitrypsine et les protéines totales sont alors mesurées, la première par un test ELISA au moyen d'anticorps de chèvre et d'anticorps de souris couplés à la peroxydase, tous deux dressés contre l'$\alpha_1$-antitrypsine humaine, les secondes par la méthode du folin. Le résultat montre que les vecteurs pULB1219, pULB1220 et pULB1221 sont 5 à 10 fois supérieurs au parent pCQV2.

**Revendications**

1. Procédé de préparation de plasmides vecteurs capables de transformer un hôte bactérien Escherichia coli et d'y promouvoir et contrôler l'expession d'un ADN hétérologue, caractérisé en ce que le plasmide comprend:

a) un promoteur partiellement synthétique de séquence identique à celle du promoteur P$_L$ du bactériophage lambda.

b) une séquence transcrite à partir du promoteur en question qui soit un leader comprenant un site de liaison des ribosomes, mise en évidence par compilation des séquences naturelles de liaison (séquence consensus), suivi d'une région riche en bases A et T dont la longueur doit être comprise entre 3 et 15 bases jusqu'à l'ATG d'initiation de la traduction.

c) une séquence de reconnaissance des enzymes de restriction KpnI, Ncol, et BamHI à cheval sur le site d'initiation de la traduction.

2. Procédé de préparation de plasmides vecteurs selon la revendication 1, caractérisé en ce que

a) le promoteur de séquence identique à celle du promoteur P$_L$ du bactériophage lambda est construit à partir d'un promoteur P$_R$ dont la partie comprise entre le site HindII situé dans la région -35 et le site d'initiation de la transcription est remplacée par un fragment synthétique de séquence identique à la séquence naturelle du P$_L$.

b) la séquence leader transcrite à partir du promoteur en question est longue de 23 bases suivies d'un codon ATG d'initiation de la traduction, elle comprend un site de liaison des ribosomes complémentaires à l'ARN 16S du ribosome sur 9 bases et situé 12 bases en amont de l'ATG.

c) les séquences reconnues par enzymes KpnI, Ncol et BamHI sont combinées en une séquence de longueur minimum qui est:

```
           KpnI                    BamHI
      ┌──────────────┐    ┌─────────────────┐
   G  G  T  A  C  C  A  T  G  G  A  T  C  C
              └────────────────┘
                    NcoI
```

3. Procédé de préparation de plasmides vecteurs selon les revendications 1 et 2 caractérisé en ce que:

a) le promoteur P_L et le leader transcrit à partir de ce promoteur sont construits à partir du promoteur P_R du plasmide vecteur pCQV2 par remplacement d'un fragment Hind-BamHI de 56 paires de bases par un fragment de 60 paires de bases synthétisé sous forme de 4 oligodésoxyribonucléotides.

b) des bactéries hôtes E.coli sont transformées par le plasmide obtenu sous a), sélectionnées, et isolées par crible caractéristique et les vecteurs portés sont caractérisés par séquençage des régions relevantes.

4. Procédé de préparation de plasmides vecteurs selon les revendications 1 à 3 caractérisé en ce que la séquence leader inventée est la suivante:

```
  A T C A G C T A A G G A G G T T T T G G T A C C A T G
  ↑                                             ↑
  Initiation de la                   initiation de la
  transcription                      traduction
```

5. Procédé de préparation de plasmides vecteurs selon les revendications 1 à 4 caractérisé en ce que le plasmide vecteur obtenu est modifié par la délétion d'un fragment HindIII-HindIII de 125 paires de bases dans le gène cl857.

6. Procédé de préparation de plasmides vecteurs selon la revendication 5 caractérisé en ce que le plasmide vecteur obtenu est modifié par l'insertion immédiatement en aval, d'une séquence qui porte les sites de restriction suivants, SacII, BalI, SalI, XhoI, SacI et PvuII, répartis sur une distance de 34 paires de bases.

7. Procédé de préparation de plasmides vecteurs selon les revendications 5 à 6 caractérisé en ce que la séquence insérée est la suivante:

```
     BamHI        SacII          BalI
   ┌────────┐ ┌─────────┐ ┌─────────────┐
   G A T C C  C G C G G  T G G C C A

     SalI          XhoI      SacI    PvuII
   ┌────────┐ ┌────────┐ ┌────────┐
   G T C G A  C T C G A  G C T C A  G
```

8. Plasmides vecteurs capables de transformer un hôte bactérien E.coli et d'y promouvoir et contrôler l'expression d'un ADN hétérologue, caractérisés en ce qu'ils sont obtenus selon les revendications 1 à 7.


**Patentansprüche**

1. Verfahren zur Herstellung von Vektorplasmiden, fähig zur Transformation von Escherichia coli-Wirtebakterien und zur Förderung und Kontrolle der Expression eines DNA-Heterologen, dadurch gekennzeichnet, daß das Plasmid umfaßt:

a) einen teilweise synthetischen Promotor mit einer Sequenz identisch zu derjenigen des Promotors P_L des Bakteriophagen lambda.

b) eine transkribierte Sequenz ausgehend von dem in Rede stehenden Promotor, der ein Leader sein soll, umfassend eine Verknüpfungsstelle der Ribosomen, dargestellt durch Aneinanderfügen der natürlichen Verknüpfungssequenzen (Sequenz consensus), gefolgt von einem Bereich reich an Basen A und T, dessen Länge zwischen 3 und 15 Basen liegen soll bis zum Start-ATG der Translation.

c) eine Erkennungssequenz der Restriktionsenzyme KpnI, NcoI und BamHI über die Startstelle der Translation.

2. Verfahren zur Herstellung von Vektorplasmiden nach Anspruch 1, dadurch gekennzeichnet, daß

a) der Promotor mit einer Sequenz identisch zu derjenigen des Promotors P_L des Bakteriophagen lambda aufgebaut wird ausgehend von einem Promotor P_R, dessen Bereich zwischen der Stelle HindII, situiert in dem Bereich -35 und der Startstelle der Transkription ersetzt wird durch ein synthetisches Fragment mit einer Sequenz identisch zur natürlichen Sequenz vom P_L.

b) die transkribierte Leader-Sequenz ausgehend von dem in Rede stehenden Promotor eine Länge besitzt von 23 Basen, gefolgt von einem ATG-Startcodon der Translation, sie eine Verknüpfungsstelle der Ribosomen komplementär zur RNA 16S des Ribosoms auf 9 Basen umfaßt und 12 Basen vor dem ATG gelegen ist,

c) die durch Enzyme KpnI, NcoI und BamHI erkannten Sequenzen kombiniert sind in einer Sequenz der Minimallänge:

```
            KpnI                    BamHI
        ┌──────────┐        ┌──────────────────┐
    G G T A C C A T G G A T C C
              └──────────────┘
                    NcoI
```

3. Verfahren zur Herstellung von Vektorplasmiden nach den Ansprüchen 1 und 2, dadurch gekennzeichnet, daß:

a) der Promotor $P_L$ und der aus diesem Promotor transkribierte Leader aufgebaut werden ausgehend von dem Promotor $P_R$ des Vektorplasmids pCQV2 durch Ersetzen eines Fragments HindII-BamHI aus 56 Basenpaaren durch ein Fragment aus 60 Basenpaaren synthetisiert in der Form von 4 Oligodesoxyribonukleotiden.

b) die Wirtebakterien E.coli durch das unter a) erhaltene Plasmid transformiert, selektioniert und durch ein charakteristisches Sieb isoliert werden und die enthaltenen Vektoren durch Sequenzierung der relevanten Bereiche charakterisiert werden.

4. Verfahren zur Herstellung von Vektorplasmiden nach den Ansprüchen 1 bis 3, dadurch gekennzeichnet, daß die erfindungsgemäße Leadersequenz die folgende ist:

```
    A T C A G C T A A G G A G G T T T T G G T A C C A T G
         ↑                                         ↑
      Start der                               Start der

      Transkription                           Translation
```

5. Verfahren zur Herstellung von Vektorplasmiden nach den Ansprüchen 1 bis 4, dadurch gekennzeichnet, daß das erhaltene Vektorplasmid modifiziert wird durch Deletion eines Fragments HindIII-HindIII aus 125 Basenpaaren in dem Gen cI857.

6. Verfahren zur Herstellung von Vektorplasmiden nach Anspruch 5, dadurch gekennzeichnet, daß das erhaltene Vektorplasmid modifiziert wird durch Insertion unmittelbar danach einer Sequenz, die die folgenden Restriktionsstellen trägt, SacII, BalI, SalI, XhoI, SacI und PvuII, verteilt über einen Bereich von 34 Basenpaaren.

7. Verfahren zur Herstellung von Vektorplasmiden nach den Ansprüchen 5 bis 6, dadurch gekennzeichnet, daß die eingeführte Sequenz die folgende ist:

```
            BamHI           SacII              BalI
        ┌──────────┐    ┌──────────────┐    ┌──────────┐
    G A T C C C G C G G T G G C C A

        SalI            XhoI        SacI      PvuII
        ┌──────────┐ ┌──────────┐ ┌──────────────┐
    G T C G A C T C G A G C T C A G
```

8. Vektorplasmide fähig zur Transformation eines Wirtebakteriums E.coli und zur Förderung und Kontrolle der Expression eines DNA-Heterologen, dadurch gekennzeichnet, daß sie nach den Ansprüchen 1 bis 7 erhalten werden.

**Claims**

1. A method of preparing vector plasmids capable of transforming a bacterial Escherichia coli host, of acting as a promoter and of controlling the expression of a heterologous DNA, characterized in that it consists of:

a) a partly synthetic promoter with a sequence identical to that of the $P_L$ promoter of the lambda bacteriophage;

b) a sequence transcribed from the promoter in question, which is a leader sequence consisting of a ribosomal binding site, manifested by the compilation of natural binding sequences (consensus sequence), followed by a region rich in A and T bases whose length must lie between 3 and 15 bases up to the translation initiation ATG;

c) a recognition sequence of KpnI, NcoI and BamHI restriction enzymes straddling the site of the initiation of the translation.

2. A method of preparing vector plasmids according to claim 1, characterized in that

a) the promoter with a sequence identical to that of the $P_L$ promoter of the lambda bacteriophage is formed from a $P_R$ promoter of which the part lying between the HindII site located in the -35 region and the site where the transcription is initiated is replaced by a synthetic fragment with a sequence identical to the natural sequence of the $P_L$;

b) the leader sequence transcribed from the promoter in question is 23 bases long, is followed by a translation initiation ATG codon and consists of one ribosomal binding site in addition to the ribosomal 16S RNA over 9 bases and located 12 bases upstream of the ATG;

c) the sequences recognized by the KpnI, NcoI and BamHI enzymes are combined into a sequence of minimum length which is:

```
        KpnI              BamHI
      ┌──────────┐ ┌──────────────────┐
      G G T A C C A T G G A T C C
              └──────────────┘
                 NcoI
```

3. A method of preparing vector plasmids according to claims 1 and 2, characterized in that:

a) the $P_L$ promoter and the leader transcribed from this promoter are formed from the $P_R$ promoter of the pCQV2 vector plasmid by the replacement of a HindII-BamHI fragment of 56 base pairs by a 60 base pair fragment synthesized in the form of oligodeoxyribonucleotides;

b) the host E. coli bacteria are transformed by the plasmid obtained under a), selected, and isolated by characteristic sieve and the vectors carried are characterized by sequencing of the relevant regions.

4. A method of preparing vector plasmids according to claims 1 to 3, characterized in that the invented leader sequence is as follows:

```
   A T C A G C T A A G G A G G T T T T G G T A C C A T G
   ↑                                                 ↑
   Initiation of                         Initiation of
   transcription                         translation
```

5. A method of preparing vector plasmids according to claims 1 to 4, characterized in that the vector plasmid obtained is modified by the removal of a HindIII-HindIII fragment of 123 base pairs in the cl857 gene.

6. A method of preparing vector plasmids according to claim 5, characterized in that the vector plasmid obtained is modified by the insertion, immediately downstream, of a sequence carrying the following restriction sites, SacII, BalI, SalI, XhoI, SacI and PvuII, distributed over a distance of 34 base pairs.

7. A method of preparing vector plasmids according to claims 5 to 6, characterized in that the inserted sequence is as follows:

```
      BamHI           SacII              BalI
   ┌──────────┐ ┌──────────────┐ ┌──────────────┐
   G A T C C C G C G G T G G C C A

      SalI            XhoI          SacI    PvuII
                              ┌──────────┐
   ┌──────────┐ ┌──────────────┐ ┌──────────────┐
   G T C G A C C T C G A G C T C A G
```

8. Vector plasmids capable of transforming a bacterial E. coli host, of acting as a promoter and of controlling the expression of a heterologous DNA, characterized in that they are obtained according to claims 1 to 7.